# EUROPÄISCHE PATENTANMELDUNG

(11) **EP 1 110 545 A2**
(43) Veröffentlichungstag der Anmeldung: **27.06.2001**
(21) Anmeldenummer: 01108141.1
(22) Anmeldetag: 10.08.1994
(51) Int. Cl.: A61K 9/52

(54) **Pharmazeutische Präparate zur gezielten Behandlung von Morbus Crohn und Colitis ulcerosa**

(30) Priorität: 01.09.1993 DE 4329503
(62) Teilanmeldung aus: 94925443.7
(71) Anmelder: Novartis AG, 4058 Basel (CH); Novartis-Erfindungen Verwaltungsgesellschaft m.b.H., 1235 Wien (AT)
(72) Erfinder: Posanski, Ulrich, 79110 Freiburg (DE)
(74) Vertreter: Becker, Konrad

(57) **Zusammenfassung**

Die Erfindung betrifft ein pharmazeutisches Präparat, das einen immunsuppressiven Wirkstoff in gelöster Form in einer mit einem oder mehreren Polymerfilmen überzogenen Stärkekapsel, Hart- oder Weichgelatinekapsel enthält.

Die Erfindung betrifft weiterhin ein Verfahren zur Herstellung des pharmazeutischen Präparats.

## Beschreibung

Die Erfindung betrifft ein pharmazeutisches Präparat zur enteralen Behandlung von Morbus Crohn und Colitis ulcerosa, welches einen immunsuppressiven Wirkstoff enthält, der zur gezielten, lokalen Wirkung im Darmbereich in Form einer besonderen galenischen Formulierung verabreicht wird, seine Verwendung und ein Verfahren zu seiner Herstellung.

Die heute bekannten Therapien zur Behandlung von Morbus Crohn und Colitis ulcerosa sind nicht sehr effektiv. Am Ende der medikamentösen Behandlung steht meistens der chirurgische Eingriff am Patienten. Es gibt zahlreiche Ansätze, die therapeutischen Möglichkeiten zu erweitern und zu verbessern, jedoch hat kein Ansatz die medizinischen Bedürfnisse bis heute optimal erfüllen können.

Eine Möglichkeit zur lokalen, enteralen Therapie der inflammatorischen Darmerkrankungen wurde mit der Entwicklung und Verwendung von speziellen Mesalazin-haltigen (5-Aminosalicylsäure) Präparaten eröffnet, die den Wirkstoff im distalen Teil des Dünndarms und im Dickdarm freisetzen. Es handelt sich bei diesen Präparaten um feste Darreichungsformen, die den Wirkstoff trotz seiner Schwerlöslichkeit in kristalliner Form enthalten.

Eine neue Möglichkeit stellt die Anwendung von immunsuppressiven Wirkstoffen dar, die wirksamer sind als die Salicylsäurederivate, jedoch bei systemischer Applikation in therapeutisch wirksamen Dosierungen erhebliche Nebenwirkungen aufweisen.

Der vorliegenden Erfindung liegt die Aufgabe zugrunde, ein pharmazeutisches Präparat zur gezielten Behandlung von Morbus Crohn und Colitis ulcerosa, das einen immunsuppressiven Wirkstoff enthält, zur Verfügung zu stellen. Das Präparat soll gezielt im Dünn- und Dickdarm wirken und eine lokale, enterale Anwendung des immunsuppressiven Wirkstoffs am Ort des inflammatorischen Erkrankungsgeschehens sicherstellen.

Überraschenderweise wird eine Verbesserung des Verhältnisses von Wirkung zu Nebenwirkungen der immunsuppressiven Wirkstoffe, d.h. eine Vergrößerung der therapeutischen Breite, durch die lokale Anwendung der Wirkstoffe in Form eines neuen pharmazeutischen Präparates erreicht, welches den Wirkstoff zum Ort des inflammatorischen Erkrankungsgeschehens transportiert und dort optimal zur Wirkung kommen läßt.

Die hier beanspruchten immunsuppressiven Wirkstoffe aus der Gruppe der Macrolide sind schwerlöslich in wäßrigen Medien, wie z.B. im Lumen des Gastrointestinaltraktes. Besonders in dem zur Wirkung des Präparates vorgesehenen Bereich des distalen Dünndarmes und des Dickdarmes sind die Auflösungsbedingungen, bedingt durch das geringe Flüssigkeitsangebot, äußerst ungünstig für schwerlösliche Wirkstoffe. Als Konsequenz sollte der Wirkstoff vorteilhafterweise in bereits gelöster Form zum Ort der Erkrankung transportiert werden. Idealerweise wird hierzu eine Lösung des Wirkstoffes in Stärkekapseln, Hart- oder Weichgelatinekapseln abgefüllt. Die native Gelatinekapsel oder Stärkekapsel übersteht den Transit durch den Magen und oberen Dünndarmbereich nicht.

Die unerwünschte Auflösung der Kapselhülle im Magen- bzw. oberen Dünndarmbereich wird durch das Überziehen der äußeren Kapselwand mit einem Polymerfilm verhindert. Die Auswahl und Verwendung geeigneter Polymeren, inclusive Zuschlagstoffe wie z.B. Weichmacher und Porenbildner, steuert den Ort der Kapselauflösung und die Freigabe der wirkstoffhaltigen Lösung.

Gegenstand der Erfindung ist ein pharmazeutisches Präparat, das einen immunsuppressiven Wirkstoff in gelöster Form in einer mit einem oder mehreren Polymerfilmen überzogenen Stärkekapsel, Hart- oder Weichgelatinekapsel enthält.

Gegenstand der Erfindung ist weiterhin ein Verfahren zur Herstellung des erfindungsgemäßen Präparats, das dadurch gekennzeichnet ist, daß der Wirkstoff in einem zur Verkapselung in Stärke- oder Gelatinekapseln geeigneten Lösungsmittel oder einem Gemisch aus mehreren Lösungsmitteln und gegebenenfalls Lösungsvermittlern und/oder anderen Hilfsstoffen gelöst wird, die Lösung in an sich bekannter Weise in Stärke-, Hart- oder Weichgelatinekapseln in abgemessener Dosis eingefüllt wird, die Kapseln verschlossen werden und die Kapseln mit einer Lösung oder Dispersion eines Polymeren oder Polymerengemisches beschichtet und getrocknet werden, wobei der Beschichtungsvorgang ein oder mehrere Male wiederholt werden kann.

Das erfindungsgemäße Präparat ist zur lokalen, enteralen Behandlung von Morbus Crohn und Colitis ulcerosa geeignet. Es enthält einen in Wasser schwerlöslichen immunsuppressiven Wirkstoff. Als Wirkstoff enthält das erfindungsgemäße Präparat Rapamycin, Tacrolimus, Cyclosporin A oder Kombinationen dieser Wirkstoffe. Zum Auflösen des Wirkstoffs sind solche Lösungsmittel geeignet, die pharmazeutisch verträglich sind und in denen sich der Wirkstoff löst.

Beispiele hierfür sind Ethanol, 1,2-Propylenglykol, Glycerin, Polyethylenglykol 300/400, Benzylalkohol, mittelkettige Triglyceride und pflanzliche Öle.

Der Lösung aus Wirkstoff und Lösungsmittel können gegebenenfalls übliche Arzneimittelhilfsstoffe, beispielsweise oberflächenaktive Mittel und Mittel zur Beeinflussung der Viskosität, zugesetzt werden. Beispiele für derartige Hilfsstoffe sind Mono-/Difettsäureglyceride, Sorbitanfettsäureester, Polysorbate, Mirj® 52, Lecithin, Natriumlaurylsulfat, Dioctylsulfosuccinat-Natrium, Cremophor® RH40/EL, Aerosil und wasserlösliche Cellulosederivate.

Es können auch Gemische der Lösungsmittel und der vorgenannten Hilfsstoffe verwendet werden. Die Wirkstoffkonzentration in dem Lösungsmittel oder Gemisch wird so eingestellt, daß sie zwischen 0,2 und 20 % (Gew./Gew.), vorzugsweise zwischen 1 und 15 % (Gew./Gew.), besonders bevorzugt zwischen 2 und 10 % (Gew./Gew.), liegt. Die Lösung des Wirkstoffes wird in einer Menge von 0,05 ml bis 2 ml, vorzugsweise in einer Menge von 0,1 bis 1,4 ml, in eine übliche Stärkekapsel, Weich- oder Hartgelatinekapsel abgefüllt. Als Gelatinekapseln oder Stärkekapseln können die verwendet werden, die üblicherweise auf pharmazeutischem Gebiet eingesetzt werden und im Handel erhältlich sind. Gegebenenfalls kann die Kapsel zusätzlich mit einer Banderole versehen werden, um zu verhindern, daß die Wirkstofflösung ausläuft. Die Herstellung der Weichgelatinekapseln erfolgt beispielsweise analog dem bekannten Scherer-Verfahren. Die Stärkekapseln sind unter dem Handelsnamen Capill® erhältlich.

Um die lokale, enterale Anwendung der immunsuppressiven Wirkstoffe Deoxyspergualin, Rapamycin, Tacrolimus und Cyclosporin A zu ermöglichen, muß nach dem Schlucken der Kapsel der Transit der intakten Kapsel durch den Magen und den oberen Dünndarmbereich gewährleistet werden. Hierzu wird die Weich- oder Hartgelatinekapsel mit einem oder mehreren Polymerfilmen überzogen, wobei die gezielte Kapselauflösung und Wirkstofffreigabe durch die Filmkomposition erreicht wird. Hierzu bieten sich zwei Prinzipien oder eine Kombination dieser Prinzipien an:
1. Die vorzeitige Kapselauflösung wird durch das Umhüllen der Kapsel mit einem Säuregruppen enthaltenden Polymerfilm verhindert, wobei der Säuretyp und die Anzahl der Säuregruppen die Auflösung des Filmes in Abhängigkeit vom pH-Wert der Umgebung steuern.
2. Die Kapselauflösung wird durch Diffusionsmechanismen gesteuert, wobei der Polymerfilm wasserunlöslich ist und die Diffusionsvorgänge durch Zuschlagstoffe, wie beispielsweise wasserlösliche Porenbildner und Weichmacher, beeinflußt werden.

Im Sinne dieser Erfindung besonders geeignete Polymere sind für das Prinzip 1:
Celluloseacetattrimellitat, -acetatsuccinat, -acetatphthalat, Hydroxypropylmethylcellulosephthalat, -acetatsuccinat, Carboxymethylethylcellulose (Handelsname z.B. Duodcell®), Polyvinylacetatphthalat (Handelsprodukte z.B. Coateric®, Opadry Enteric®), Copolymerisate aus Vinylacetat und Crotonsäure (Handelsprodukt z.B. Coating CE 5142®), Polymethacrylate, wie z.B. Copolymerisate aus Methacrylsäure und Methylmethacrylat, Copolymerisate aus Methacrylsäure und Ethylacrylat (Handelsprodukte sind z.B. Eudragit® L/L30D). Es können auch Mischungen dieser Polymeren verwendet werden.
Geeignete Polymere für das Prinzip 2 sind:
Methylcellulose (Handelname z.B. Methocel), Ethylcellulose (Handelsprodukt z.B. Ethocel®, Aquacoat® ECD30), Celluloseacetat, Celluloseacetatpropionat, Celluloseacetatbutyrat, Polyvinylderivate, wie z.B. Polyvinylalkohol, Polyvinylacetat, Vinylacetat/Vinylpyrrolidon-Copolymere (Handelsprodukte sind z.B. PVP-VA-Typen der GAF), Copolymerisate aus Methacrylsäure und Ethylacrylat (Handelsprodukte sind z.B. Eudragit® RL/RS/NE30D/RL30D/RS30D), Copolymerisate aus Polymethylvinylether und Malonsäureanhydrid, Copolymerisate aus Polymethylvinylether und Malonsäure oder deren Ethyl-, Isopropyl-, n-Butylestern (Handelsprodukte sind z.B. die Gantrez®-Polymeren der Serien ES/AN/S), oder Mischungen dieser Polymeren.

Für beide Prinzipien ist darüberhinaus die Dicke des Polymerfilmes auf der Kapseloberfläche für den Verlauf der Kapselauflösung und die Wirkstofffreigabe von Bedeutung. Die notwendige Filmdicke kann für jedes Polymere individuell verschieden sein. Zusätzlich hängt sie von anderen Hilfsstoffen, wie z.B. Weichmachern, dem während des Befilmungsprozesses verbrauchten Lösungs- oder Dispersionsmittel, der Filmauftragstechnik und der Kapselform ab. In der Praxis liegen die Auftragsmengen zwischen 1 mg/cm² und 100 mg Filmtrockensubstanz / cm² Kapseloberfläche. Der Gewichtsanteil des getrockneten Filmüberzuges an der gesamten Arzneiform liegt in der Regel unterhalb von 10 % (Gew.).

Werden beide Prinizipien in Kombination benutzt und die Polymeren getrennt aufgetragen, dann dient ein Film entsprechend dem Prinzip 1 als äußerer Film. Dieser äußere Film muß eine Auflösungszeit von mehr als 2 Stunden in Magensaft aufweisen.

Die Eigenschaften der Polymerfilme können weiterhin durch Zusätze von Porenbildnern und Weichmachern beeinflußt werden. Geeignete Porenbildner zur Bildung von offenen Poren und damit zur Steigerung der Diffusionsrate durch den Polymerüberzug sind wasserlösliche Stoffe, wie z.B. Lactose, Saccharose, Sorbit, Mannit, Glycerin, Polyethylenglykol bestehend aus weniger als 6000 Ethylenoxideinheiten, 1,2-Propylenglykol, Hydroxypropylcellulose, Hydroxypropylmethylcellulose, Methylcellulose sowie deren Mischungen. Der Gewichtsanteil der Porenbildner am getrockneten Filmüberzug ist kleiner als 20 % (Gew./Gew.).

Als Weichmacher sind geeignet die Alkylester der Zitronensäure, Weinsäure und 1,8-Octandicarbonsäure, wie z.B. Triethylcitrat, Tributylcitrat, Acetyltriethylcitrat, Dibutyltartrat, Diethylsebacat, bzw. Ester der Phthalsäure, wie z.B. Dimethylphthalat, Diethylphthalat, Dioctylphthalat, bzw. Glycerinester, wie z.B. Rizinusöl, Sesamöl, acetylierte Fettsäureglyceride, Glycerintriacetat, Glycerindiacetat, bzw. höhere Alkohole, wie z.B. Glycerin, 1,2-Propylenglykol, bzw. Polyether, wie z.B. Polyethylenglykole und Polyoxyethylen-Polypropylen-Blockcopolymere, bzw. Netzmittel, wie z.B. PEG-400-Stearat, Sorbitanmonooleat, PEG-Sorbitanmonooleat.

Das Polymere oder ein Gemisch aus Polymeren wird zum Auftragen in einem organischen Lösungsmittel bzw. in einem Lösungsmittelgemisch aufgelöst oder dispergiert. Als Lösungsmittel sind beispielsweise Ethanol, Isopropanol, n-Propanol, Aceton, Ethylacetat, Methylethylketon, Methanol, Methylenchlorid, tert.-Butanol, Propylenglykolmonomethylether und Wasser geeignet.

Es können auch Lösungsmittelgemische oder Gemische dieser Lösungsmittel mit Wasser verwendet werden. Zur besseren Verarbeitung der Filmüberzüge können diesen übliche Hilfsstoffe, wie beispielsweise kolloidales Siliciumdioxid, Talkum und Magnesiumstearat, zugesetzt werden. Zum Auftragen und Trocknen des Polymerfilms sind alle bekannten Verfahren zur Tabletten- oder Pelletbefilmung, wie z.B. das Tauchschwert-, Tauchrohr-, Dragier-, Wirbelschicht-, Wurster-, Accela-Cota-, Hi-Coater-, Driacoater- oder Kugelcoaterverfahren, geeignet.

Der Fachmann kann durch einfache Vorversuche die Eigenschaften der Polymerfilme feststellen. Es ist hierbei von wesentlicher Bedeutung, daß nach dem Schlucken des Präparats der Transit der intakten Kapsel durch den Magen und den oberen Dünndarmbereich gewährleistet wird.

Beispielsweise werden die überzogenen Kapseln einer Prüfung der Wirkstofffreisetzung in einer "Dissolution-Rate"-Test-apparatur mit Drehkörbchen nach USP unterworfen. Hierzu werden die Kapseln zunächst für zwei Stunden künstlichem Magensaft ausgesetzt. Danach wird das Medium auf künstlichen Darmsaft pH 6,8 umgestellt. Nach weiteren vier Stunden wird auf künstlichen Darmsaft pH 7,2 eingestellt. Während der gesamten Versuchsdauer wird die Wirkstoffkonzentration im Medium, z.B. mit einer geeigneten HPLC-Methode, fortlaufend bestimmt. Der Wirkstoff sollte während der Versuchsperioden in künstlichem Magensaft und in künstlichem Darmsaft pH 6,8 nicht detektierbar sein, ansonsten könnte der Transit der intakten Kapsel zum Wirkort nicht gewährleistet werden.

Die folgenden Beispiele erläutern die Erfindung, ohne sie einzuschränken.

### Beispiel 1

Die folgende Wirkstofflösung wird in ovale Weichgelatinekapseln der Größe 5 minims gefüllt:

Angaben in mg/Kapselinhalt nach der Herstellung:

| | |
|---|---|
| Cyclosporin A | 25,0 |
| Ethanol 96%ig | 25,0 |
| Polyoxyethylen-(40)-hydriertes Rizinusöl | 87,5 |
| Neutralöl | 50,0 |
| Di/Tri/Tetraglycerolfettsäureester | 62,5 |

Auf die getrockneten Weichgelatinekapseln werden nachfolgend zwei Polymerfilme aufgetragen. Hierzu werden 250.000 Weichgelatinekapseln in eine Accela-Cota (AC48) gefüllt. Von der ersten Polymerlösung werden 15,0 kg aufgesprüht und das Lösungsmittel wird während des Prozesses kontinuierlich entfernt. Die Zulufttemperatur zum Abtrocknen der Kapseln beträgt 35°C.

Die erste Polymerlösung besteht aus (Angaben in Gew.-%):

| | |
|---|---|
| Ethylcellulose | 5,0 |
| Triacetin | 1,0 |
| Polyethylenglykol 1500 | 0,6 |
| Ethanol 96%ig | 93,4 |

Die zweite Polymerlösung besteht aus (Angaben in Gew.-%):

| | |
|---|---|
| Hydroxypropylmethylcellulosephthalat (HP-55) | 8,0 |
| Dest. acetylierte Glyceride | 0,8 |
| Aceton | 46,0 |
| Ethanol 96%ig | 45,2 |

13,5 kg der zweiten Lösung werden in der gleichen Apparatur aufgetragen. Die Zulufttemperatur wird auf 30°C abgesenkt. Nach Abschluß des Filmauftrages wird die Temperatur zum Entfernen von Restlösungsmitteln auf 40°C angehoben.

### Beispiel 2

Die folgende Lösung von Tacrolimus wird in Weichgelatinekapseln der Größe 8 minims gefüllt:

Angabe des Kapselinhaltes in mg pro Kapsel nach der Herstellung:

| | |
|---|---|
| Tacrolimus | 10,0 |
| Polysorbat 80 | 270,0 |
| Neutralöl | 135,0 |
| Sorbitanmonolaurat | 45,0 |

Auf die getrockneten Kapseln wird ein Celluloseacetatfilm aufgetragen. Hierzu werden 150.000 Kapseln in einen Driacoater (DR 1200) mit Lösungsmittelrückgewinnungssystem gefüllt. Die Zulufttemperatur wird während der Befilmung auf 30°C eingestellt. Die aufgesprühte Polymerlösung hat die folgende Komposition :

| | |
|---|---|
| Celluloseacetat | 5,0 |
| Polyethylenglykol 4000 | 0,3 |
| Aceton | 80,0 |
| Wasser | 14,7 |

### Beispiel 3

Eine Lösung mit dem Wirkstoff Rapamycin wird in Hartgelatinekapseln der Größe "1" abgefüllt. Die Angaben entsprechen dem Kapselinhalt in mg.

| | |
|---|---|
| Rapamycin | 5,0 |
| Polysorbat 60 | 230,0 |
| Polyethylenglykol 400 | 190,0 |
| Glycerol | 5,0 |
| 1,2-Propylenglykol | 20,0 |
| Gesamtinhalt | 450,0 |

Hinzu kommt das Leergewicht der Kapsel von 77 mg. Auf diese gefüllten Hartgelatinekapseln werden nacheinander drei Polymerlösungen aufgetragen (Angaben jeweils in Gew.-%).

Die Komposition der ersten Lösung:

| | |
|---|---|
| Hydroxypropylcellulose | 7,5 |
| Polyethylenglykol 1500 | 0,5 |
| Ethanol 96%ig | 92,0 |

Dieser Polymerfilm wird zum verschließen der Kapseln aufgetragen und dient den weiteren Verarbeitungsstufen (Under-coat).

Die Komposition der zweiten Lösung:

| | |
|---|---|
| Eudragit RS 30D | 27,0 |
| Eudragit RL 30D | 6,0 |
| Triethylcitrat | 2,0 |
| Talkum | 2,5 |
| Wasser | 62,5 |

Die Komposition der dritten Lösung:

| | |
|---|---|
| Hydoxypropylmethylcelluloseacetatsuccinat | 10,0 |
| Triethylcitrat | 3,0 |
| Talkum | 3,0 |
| Wasser | 84,0 |

Die Befilmung erfolgt in einer Wirbelschichtapparatur. Die Zulufttemperatur liegt während der Befilmung bei 48°C. Die Sprührate und der Luftdurchsatz werden so eingestellt, daß die Ablufttemperatur bei 21°C bleibt.

## Patentansprüche

1. Pharmazeutisches Präparat, das einen immunsuppressiven Wirkstoff in gelöster Form in einer mit einem oder mehreren Polymerfilmen überzogenen Stärkekapsel, Hart- oder Weichgelatinekapsel enthält.

2. Pharmazeutisches Präparat nach Anspruch 1, dadurch **gekennzeichnet**, daß es als Wirkstoff Rapamycin, Tacrolimus, Cyclosporin A oder Kombinationen dieser Wirkstoffe enthält.

3. Pharmazeutisches Präparat nach Anspruch 1 oder 2, dadurch **gekennzeichnet**, daß der Kapselüberzug aus einem Polymer aus der Gruppe der Cellulosederivate, der Methacrylsäurederivate, der Polyvinylderivate oder deren Mischungen besteht.

4. Pharmazeutisches Präparat nach Anspruch 1, 2 oder 3, dadurch **gekennzeichnet**, daß die Hart- oder Weichgelatinekapsel mit einem Polymerfilm oder mehreren Polymerfilmen aus Methyl-, Ethylcellulose, Celluloseacetat, Celluloseacetatbutyrat, Celluloseacetatphthalat, Celluloseacetattrimellitat, Celluloseacetatpropionat, Hydroxypropylmethylcellulose, Hydroxypropylcellulose, Hydroxypropylmethylcellulosephthalat, Hydroxypropylmethylcelluloseacetatsuccinat, Polyvinylalkohol, Polyvinylacetat, Polyvinylacetatphthalat, Methacrylsäure-Methylmethacrylat-Copolymeren, Methacrylsäure-Ethylacrylat-Copolymeren, Polymethylvinylether-Malonsäureanhydrid-Copolymerisaten, Polymethylvinylether-Malonsäure-Ethyl, -Isopropyl, -n-Butylester-Copolymerisaten oder deren Mischungen überzogen sind.

5. Pharmazeutisches Präparat nach mindestens einem der Ansprüche 1 bis 4, dadurch **gekennzeichnet**, daß die Wirkstoffkonzentration (w/w) in der verkapselten Lösung zwischen 0,2 und 20 %, vorzugsweise zwischen 1 und 15 %, besonders bevorzugt zwischen 2 und 10 %, liegt.

6. Pharmazeutisches Präparat nach mindestens einem der Ansprüche 1 bis 5, dadurch **gekennzeichnet**, daß der gelöste Wirkstoff im Dünn- und/oder Dickdarmbereich des Menschen aus der Kapsel freigesetzt wird.

7. Pharmazeutisches Präparat nach einem der Ansprüche 1 bis 6 zur Behandlung von Morbus Crohn und Colitis ulcerosa.

8. Verfahren zur Herstellung eines pharmazeutischen Präparats nach einem der Ansprüche 1 bis 6, dadurch **gekennzeichnet,** daß der Wirkstoff in einem zur Verkapselung in Stärkekapseln oder Gelatinekapseln geeigneten Lösungsmittel oder einem Gemisch aus mehreren Lösungsmitteln und anderen Hilfsstoffen gelöst wird, die Lösung in an sich bekannter Weise in Stärkekapseln, Hart- oder Weichgelatinekapseln in abgemessener Dosis eingefüllt wird, die Kapseln verschlossen werden und die Kapseln mit einer Lösung oder Dispersion eines Polymeren oder Polymerengemisches beschichtet und getrocknet werden, wobei der Beschichtungsvorgang ein oder mehrere Male wiederholt werden kann.
